Europäisches Patentamt

European Patent Office

Office européen des brevets

(19)

(11) Publication number: **0 132 983 B2**

(12) # NEW EUROPEAN PATENT SPECIFICATION

(45) Date of publication of the new patent specification: **12.06.91 Bulletin 91/24**

(51) Int. Cl.⁵: **C09H 9/04,** C09H 7/00, C08J 3/09

(21) Application number: **84304815.8**

(22) Date of filing: **13.07.84**

(54) **Production of gelatin spherical gels and their use.**

(30) Priority: **14.07.83 JP 128462/83**
**20.04.84 JP 80772/84**

(43) Date of publication of application:
**13.02.85 Bulletin 85/07**

(45) Publication of the grant of the patent:
**07.10.87 Bulletin 87/41**

(45) Mention of the opposition decision:
**12.06.91 Bulletin 91/24**

(84) Designated Contracting States:
**CH DE FR GB LI**

(56) References cited:
**DD-C- 2 005 923**

(56) References cited:
**DE-A- 1 445 105**
**DE-C- 813 188**
**FR-A- 2 271 270**
**JP-A-55 047 130**

(73) Proprietor: **Hitachi Chemical Co., Ltd.**
**1-1, Nishishinjuku 2-chome**
**Shinjuku-ku, Tokyo 160 (JP)**

(72) Inventor: **Hirai, Osamu**
**Yamazakiryo 2-9 Higashicho-4-chome**
**Hitachi-shi (JP)**
Inventor: **Mukoyama, Yoshiyuki**
**20-5, Nishinarusawacho-3-chome**
**Hitachi-shi (JP)**

(74) Representative: **Goldin, Douglas Michael et al**
**J.A. KEMP & CO. 14, South Square Gray's Inn**
**London WC1R 5EU (GB)**

EP 0 132 983 B2

## Description

This invention relates to a process for producing gelatin spherical gels, and more particularly to a process for producing gelatin spherical gels suitable for an embolic agent or a carrier for impregnating pharmaceuticals.

In recent years, studies on embolization of arteriae, selective intra-arterial injection of drugs, and the like have been made extensively in the field of radiology. These studies have their objects to conduct embolization on a trophic arteria having neoplasm as pretreatment of surgical removal of the neoplasm so as to reduce the bleeding amount during operation, or to reduce the neoplasm by closure of tropic arteria or by selective injection of pharmaceuticals from tropic arteria in the case of carcinomatous neoplasm when radical operation is impossible.

On the other hand, embolic agents can be divided into two groups depending on purposes, that is, those having ephemeral embolism or having solubility, and those having permanent embolism or having insolubility. As embolic agents for such purposes, there are used those obtained by dissolving gelatin in water, foaming, subjecting to lyophilization to form spongy body and cutting into several millimeters at a side or powdered ; those obtained by adding formaldehyde to gelatin, conducting polymerization with heating, blowing air bubbles thereinto to form spongy body and cutting into several millimeters at a side or powdered ; or the like. In the case of selective injection of pharmaceuticals, these substances are impregnated with pharmaceuticals before use. But since these substances are in broken form, they have a defect in that they are difficult to adhere to blood vessels compared with spherical ones.

As spherical substances available commercially, there are known those made from organic polymers such as polystyrenes, poly(acrylic acids ester)s, polyvinyl alcohols, those made from inorganic substances such as silica, glass, etc. But these substances have a problem from the viewpoint of safety when they are included in a living body.

At present, as the embolic agent, there is used gelatin sponge cut finely by aknife or the like. But this requires complicated procedures to obtain a necessary size and does not produce spherical form, which results in not only being insufficient in embolic effect but also making a catheter treatment procedure difficult. Further, it is also pointed out that when gelatin sponge is exposed to working circumstances for a long peroid of time, there is a danger of contamination (C.A. Athanasonlis, R. C. Pfister, R.E. Green : "Interventional Radiology", W.B. Sauders Co., 1982).

It is an object of this invention to provide spherical substances usuable as embolic agent, etc and a process for producing the same overcoming the problem mentioned above.

This invention provides a process for producing gelatin spherical gels which comprises dispersing an aqueous solution of gelatin and an aliphatic dial or a water-soluble polyvalent epoxide in a dispersing medium obtained by dissolving water-insoluble ethyl cellulose in a non-polar alicyclic hydrocarbon solvent which is not miscible with water, and conducting a crosslinking reaction.

This invention also provides crosslinked gelatin spherical gels (particles) having a particle size of 50 to 1000 μm for use as an embolic agent.

Gelatin has various molecular weights depending on raw materials used. In this invention, there is no particular limit to the molecular weight. Gelatin has functional groups such as $-NH_2$, $-OH$ and $-COOH$ which become reactive sites at the crosslinking reaction.

The aliphatic acid or polyvalent epoxide undergoes a crosslinking reaction with gelation and is hereinafter referred to as "crosslinking agent". Examples of aliphatic dials include glyoxal, propanedial, butanedial, pentanedial (glutaraldehyde), and hexanedial ; water soluble polyvalent expoxides include ethylene glycol diglycidyl ether, polyethylene glycol diglycidyl ether, glycerol polyglycidyl ether, diglycerol polyglycidyl ether, sorbitol polyglycidyl ether and diglycidyl methyl-hydantoin.

These crosslinking agents can be used alone or as a mixture thereof, preferably in an amount of 1/20 to 20 equivalent weight, more preferably 1/10 to 10 equivalent weight, per equivalent weight of the group in the gelatin reactive with the crosslinking agent. The group in the gelatin reactive with a dial is an amino ($NH_2$) group and that reactive with an epoxide is an amino ($NH_2$) and carboxyl ($COOH$) groups. When the amount of crosslinking agent is too small, the degree of crosslinking is lowered to easily break the spherical form, whereas the amount of crosslinking agent is too large, the degree of crosslinking is also lowered not only to easily break the spherical form but also to retain unreacted functional groups of the crosslinking agent in the resulting particles due to the reaction of the crosslinking agent in the grafted form. As the crosslinking agent, it is preferable to use those which do not react with ethyl cellulose.

As the dispersing medium, there is used one obtained by dissolving water-insoluble ethyl cellulose in a non-polar hydrocarbon solvent which is not miscible with water. This solvent can dissolve the water-insoluble ethyl cellulose. The non-polar alicyclic hydrocarbon solvent can be used together with one or more polar solvents such as esters, ketones, halogenated alkanes, ethers, alcohols or aromatic hydrocarbons, these being unreactive with the crosslinking agent.

Preferable examples of the alicyclic hydrocarbons are those having 5 to 10 carbon atoms or alkyl substituted alicyclic hydrocarbons such as cyclopentane, cyclohexane, cycloheptane, methycyclohexane, cyclooctane, decalin. These alicyclic hydrocarbons can be used alone or as a mixture thereof.

The aromatic hydrocarbons include aromatic hydrocarbons and halogen substituted aromatic hydrocarbons having 6 to 8 carbon atoms Examples of these aromatic hydrocarbons are benzene, toluene, zylenes, ethylbenzene, chlorobenzene, dichlorobenzene, bromobenzene, dibromobenzene. These can be used alone or as a mixture thereof.

As the esters, there can preferably be used these obtained from fatty acids with 1 to 8 carbon atoms or aromatic carboxylic acids with 7 to 8 carbon atoms and alcanols having 1 to 8 carbons alone or as a mixture thereof. Examples of the esters are methyl acetate, ethyl acetate, n-butyl acetate, benzyl acetate, methoxyethyl acetate, methyl caproate, methyl benzoate, diethyl phthalate.

As the ketones, there can preferably be used aliphatic ketones having 5 to 8 carbon atoms, and aromatic ketones having 8 to 13 carbon atoms alone or as a mixture thereof.Examples of the ketones are methyl isobutyl ketone, cyclohexanone, methyl amyl ketone, hexyl methyl ketone,acetophenone, benzophenone.

As the halogenated alkanes, there can preferably be used alkanes having 1 to 4 substituted halogens alone or as a mixture thereof. Examples of the halogenated alkanes are methylene chloride, carbon tetrachloride, 1,2-dichloroethane, 1,1,2-trichloroethane, pentachloroethane, 1,2-dichloropropane, 1,2-dichlorobutane, 1,2-dibromoethane.

As the ethers, there can preferably be used straight-chain or cyclic ethers having 4 to 8 carbon atoms alone or as a mixture thereof. Examples of the ethers are di-n-propyl ether, di-n-butyl ether, tetrahydrofuran, dioxane, diethylene glycol dimethyl ether.

As the alcohols, there can preferably be used alkanols having 4 to 8 carbon atoms or alkoxy alkanols having 4 to 8 carbon atoms alone or as a mixture thereof. Examples of the alcohols are n-butanol, n-octanol, methoxyethanol, ethoxyethanol, butoxyethanol, diethylene glycol monomethyl ether.

As the water-insoluble ethyl cellulose, there can be used those having an ethoxy group content of 43 to 50%. The words "ethoxy group content" mean a weight percent of the ethoxy group in ethyl cellulose. Among those mentioned above, those having relatively smaller ethoxy group content, for example those having 43 to 46% by weight of the ethoxy group content, are hardly soluble in the above-mentioned non-polar alicyclic hydrocarbon solvent. Therefore, when the above-mentioned non-polar alicyclic hydrocarbon solvent is used alone as the dispersing medium, it is preferable to use those having 47 to 50% by weight of the ethoxy group content.

When the water-insoluble ethyl cellulose is used in combination with one or more above-mentioned non-polar alicyclic hydrocarbon solents there can be formed a dispersing medium. But since the solubility of ethyl cellulose in a non-polar alicyclic hydrocarbon solvent is poor as mentioned above, it is preferable to use the above-mentioned polar solvent or aromatic hydrocarbon together as dispersing medium.

When a mixture of non-polar alicyclic hydrocarbon solvent and at least one aromatic hydrocarbon is used as dispersing medium, the aromatic hydrocarbon can be used in an amount of preferably 400% by weight or less, more preferably 200% by weight or less based on the weight of the non-polar organic solvent.

When a mixture of non-polar organic solvent and at least one of the esters, ketones and halogenated alkanes is used as dispersing medium, the latter component can be used in an amount of preferably 200% by weight or less, more preferably 150% by weight or less, based on the weight of the non-polar alicyclic hydrocarbon solvent.

When a mixture of non-polar alicyclic hydrocarbon solvent and at least one of the ethers and alcohols is used as dispersing medium, the latter component can be used in an amount of preferably 30% by weight or less, more preferably 20% by weight or less, based on the weight of the non-polar alicyclic hydrocarbon solvent.

When the aromatic hydrocarbons, ketones, esters, halogenated alkanes, ethers, alcohols are used together with the non-polar alicyclic hydrocarbon solvent in amounts larger than the values mentioned above, there is a tendency not to provide a good dispersing medium.

The water-insoluble ethyl cellulose can be used in an amount of preferably 0.05 to 10% by weight, more preferably 0.5 to 5% by weight, based on the total weight of the dispersing medium.

It is preferable to use the dispersing medium in an amount of preferably 50 to 2000% by weight, more preferably 100 to 1000% by weight, based on the total weight of gelatin, the crosslinking agent and water (hereinafter referred to as "the amount of water phase"). When the amount of the dispersing medium is too much, the productivity becomes poor, while when the amount of the dispersing medium is too small, the stability of dispersing medium becomes poor.

The amount of gelatin can be selected optionally from 5% by weight to the saturated concentration based on the weight of the water phase. If the amount of gelatin is too small the productivity is lowered.

The dispersion of an aqueous solution of gelatin and the crosslinking agent in the dispersing medium can be conducted by adding an aqueous solution obtained by dissolving gelatin and the crosslinking agent in water to a liquid medium immiscible with water, followed by stirring. But since gelatin easily reacts with the crosslinking agent, it is preferable to disperse an aqueous solution of gelatin in the liquid immiscible with water, followed by addition of the crosslinking agent or an aqueous solution thereof thereto.

The crosslinking reaction can be conducted in the dispersed state at a temperature of from room temperature to the boiling point of water or the dispersing medium for, for example, 5 to 10 minutes.

As stirring method during the reaction, there can be employed a stirring method by using an emulsator accompanying high-speed shearing, a stirring method by using a marine propeller stirrer or magnetic stirrer not accompanying mechanical cutting or grinding, and the like. These stirring methods can be selected depending on the desired particle size. When the stirring with high-speed shearing is employed, it is preferable to conduct such a stirring before the crosslinking reaction so as not to destroy crosslinked particles.

The thus obtained polypeptide spherical gels can be recovered by filtration or decantation, washing with a solvent having a relatively low boiling point for purification, followed by drying under atmospheric or reduced pressure.

If a very limited range of particle size is required, a conventional classification can be employed.

The spherical gelatin particles have a particle size of preferably 50 to 1000 μm (in dry state) and a swelling volume of preferably 5 to 100 ml/g when measured in an isotonic sodium chloride solution. The swelling volume shows a degree of crosslinking.

When the spherical gelatin particles are used as embolic agent, it is preferable to use those having a particle size of 100 to 1000 μm in dry state and a swelling volume of 5 to 100ml/g when measured in an isotonic sodium chloride solution. The swelling volume in the isotonic sodium chloride solution of 5 to 60 ml/g is more preferable and that of 5 to 30 ml/g is the most preferable.When the particle size of the crosslinked spherical gelatin particles is too small, there often arises a fear of embolizing capillarys at normal portions while passing through the portion to be embolized. When the swelling volume (th degree of swelling) is too small, the embolic agent becomes rigid, is difficult to be injected into blood vessels due to lack of flexibility, and has a fear of causing inflammation at an inner wall of the embolized portion. On the other hand, when the degree of swelling is too large, the embolic agent swells in a contrast medium or causes agglomeration of individual particles, which results in making the injection difficult and making effective embolization difficult.

In this invention, the swelling volume is determined by placing 1 g of the particles in a messcylinder, adding an isotonic sodium chloride solution to the messcylinder sufficiently, allowing the messcylinder to stand day and night, and measuring the volume of sedimented swollen particles.

The embolic agent according to this invention can be injected into a blood vessel, for example, by a Seldinger technique, wherein a catheter is inserted into a desired portion which is supplied the embolic agent through the catheter. In such a case, it is preferable to use the embolicagent dispersed in and swelled with a contrast medium. The embolic agent should be well dispersed in the contrast medium so as not to cause agglomeration of individual particles. For such a purpose, it is preferableto use the contrast medium 2 to 10 times as large as the swelling volume.

The contrast medium can be obtained by dissolving a contrast sensitivity component in a liquid medium which is safe to a living body and does not interfere contrast. The concentration of the contrast sensitivity component is not particularly limited and can be 100% when a contrast sensitivity component is liquid and has a sufficiently low viscosity. Examples of the contrast sensitivity component are meglumine sodium amidotrizoate, meglumine amidotrizoate, meglumine iotalamate, meglumine sodium iodamide, meglumine iodamide, metrizamide, L(+) – N,N' – bis – (2 – hydroxy – 1 – hydroxymethylethyl) – 2, 4, 6–triiodo – 5 – lactamide-isophthalamide, etc. These can be used in a suitable concentration, usually as a 30 to 80% by weight solution.

It is preferable to use a contrast medium which has a little or no difference in specific gravity compared with the crosslinked spherical gelatin particles. Since the specific gravity of the crosslinked spherical gelatin particles is about 1.3, the specific gravity of contrast medium is preferably about 1.2 to 1.4. Among contrast sensitivity components mentioned above, meglumine sodium amidotrizoate is more preferable considering the contrast ability and adjustment of the specific gravity in the range of 1.2 to 1.4.

This invention is illustrated by way of the following Examples and Comparative Examples.

Example 1

In a dispersing medium comprising 150 g of cyclohexane and 50 g of toluene, 6 g of ethyl cellulose (the ethoxy group content : 49% by weight) was dissolved. The resulting dispersing medium was introduced into a 500-ml flask equipped with a cooling tube and agitating blades made from polytetrafluoroethylene. The stirring speed was controlled at 400 r.p.m and the temperature was raised to70°C. Then, 40 g of an aqueous solution of gelatin obtained by dissolving gelatin in water in a concentration of 30% by weight was added to the flask, followed by addition of 4 g of a 50% aqueous solution of glutaraldehyde (4 equivalent weight of glutaraldehyde per equivalent weight of the amino group of gelatin) to the flask. The reaction was conducted at 70°C for 5 minutes to give brown particles. The resulting particles were collected by filtration, washed with ethyl acetate, followed by washing with acetone. The particles had a spherical form and a particle size of 0.1 to 1 mm when observed by a microscope.

Example 2

In a dispersing medium comprising 150 g of cyclohexane and 50 g of toluene, 6 g of ethyl cellulose (the ethoxy group content : 49% by weight) was dissolved. The resulting dispersing medium was introduced into a 500-ml flask equipped with a cooling tube and agitating blades made from polytetrafluoroethylene. The stirring speed was controlled at 400 r.p.m and the temperature was raised to 70°C. Then, 40 g of an aqueous solution of gelatin obtained by dissolving gelatin in water at 50°C in a concentration of 30% by weight was added to the flask, followed by addition of 0.15 g of a 50% aqueous solution of glutraaldehyde (0.15 equivalent weight of glutaraldehyde per equivalent weight of the amino group of gelatin) to the flask. The reaction was conducted at 70°C for 5 minutes to give brown particles. The resulting particles were collected by filtration, washed with ethyl acetate, followed by washing with acetone. The particles had a spherical form and a particle size of 0.1 to 1 mm when observed by a microscope. The resulting particles were classified to collect those having a particle size of 300-350 μm, which had the degree of swelling of 21.2 ml/g when measured in an isotonic sodium chloride solution.

Example 3

In a dispersing medium comprising 150 g of decalin and 50 g of toluene, 6 g of ethyl cellulose (the ethoxy group content : 49% by weight) was dissolved. The resulting dispersing medium was introduced into a 500-ml flask equipped with a cooling tube and agitating blades made from polytetrafluoroethylene. The stirring speed was controlled at 400 r.p.m and the temperature was raised ot 70°C. Then, 40 g of an aqueous solution of gelatin obtained by dissolving gelatin in water at 50°C in aconcentration of 30% by weight was added to the flask, followed by addition of 4 g of a 50% aqueous solution of glutaraldehyde (4 equivalent weight per equivalent weight of the amino group of gelatin) to the flask. The reaction was conducted at 70°C for 5 minutes to give brown particles. The resulting particles were collected by filtration, washed with ethyl acetate, followed by washing with acetone. The particles had aspherical form and a particle size of 0.1 to 1 mm when observed by a microscope.

Example 4

In a dispersing medium comprising 150 g of cyclohexane and 50 g of ethyl acetate, 6 g of ethyl cellulose (the ethoxy group content : 49% by weight) was dissolved. The resultingdispersing medium was introduced into a 500-ml flask equipped with a cooling tube and agitating bladesmade from polytetrafluoroethylene. The stirring speed was controlled at 400 r.p.m and thetemperature was raised to 70°C. Then, 40 g of an aqueous solution of gelatin obtained by dissolving gelatin in water at 50°C in a concentration of 30% by weight was added to the flask, followed by addition of 4g of a 50% aqueous solution of glutaraldehyde (4 equivalent weight per equivalent weight of the amino group of gelatin) to the flask. The reaction was conducted at 70°C for 5 minutes to give brown particles.The resulting particles were collected by filtration, washed with ehtyl acetate, followed by washing with acetone. The particles had a spherical form and a particle size of 0.1 to 1 mm when observed by a microscope.

Comparative Example 1

In 200 g of cyclohexane, 6 g of polyoxyethylene nonylphenyl ether as dispersing agent was dissolved. The resulting solution was introduced into a 500-ml flask equipped with a cooling tube and agitating blades made from polytetrafluoroethylene. The stirring speed was controlled at 400 r.p.m. and the temperature was raised to 70°C. Then, 40 g of an aqueous solution of gelatin obtained by dissolving gelatin in water at 50°C in a concentration of 30% by weight was added to the flask, followed by addition of 4 g of a 50% aqueous solution of glutaraldehyde to the flask. The reaction was conducted at 70°C for 5 minutes. As a result, gelatin in block form, not in the spherical form, was obtained.

## Comparative Example 2

In 200 g of toluene, 6 g of ethyl cellulose (the ethoxy group content : 49% by weight) was dissolved. The resulting solution was introduced into a 500-ml flask equipped with a cooling tube and a stirring rod made from polytetrafluoroethylene. The stirring speed was controlled at 400 r.p.m andthe temperature was raised to 70°C. Then, 40 g of an aqueous solution of gelatin obtained by dissolving gelatin in water at 50°C in a concentration of 30% by weight was added to the flask, followed by addition of 4 g of a 50% aqueous solution of glutaraldehyde to the flask. The reaction was conducted at 70°C for 5 minutes. As a result, gelatin in block form, not in the spherical form, was obtained.

## Examples 5 to 9

The procedure of Example 1 was repeated except for changing the using amount of the 50% aqueous solution of glutaraldehyde as shown in Table 1 to obtain cross-linked spherical particles classified so as to have a particle size of 300 to 350 μm. Swelling volumes in an isotonic sodium chloride solution of individual particles are also shown in Table 1.

TABLE 1

| Example No. | Using amount of 50% glutaraldehyde aq. soln. (g) | Swelling volume (ml/g) |
|---|---|---|
| 5 | 0.29 | 9.8 |
| 6 | 0.62 | 7.8 |
| 7 | 1.3 | 6.9 |
| 8 | 2.7 | 5.9 |
| 9 | 6 | 6.5 |

## Application Example

Slurries were prepared by dispersing 0.5 g of the crosslinked spherical gelatin particles obtained in Examples 2 and 5 to 9 in 20 ml of meglumine sodium amidotrizoate injection (trade name ANGIOGRAFIN manufactured by Schering AG).

Using each slurry thus prepared, the following test was conducted. Dogs were used for the test, since dogs have a fibrin decomposition system characterized by rapid repassing through an embolized arteria as well as an increase of plasmin growth and short U-globulin dissolution time compared with human being. A dog was given 50 to 100 mg/kg of ketamine at a muscle, followed by injection of 0.5 cc/kg of pentobarbital at a vein to anesthetize the dog. Then a catheter was introduced into a kidnetyof the dog by the Seldinger technique, followed by injection of the slurry through the catheter until 500 mg of the gelatin spherical particles were fed so as to embolize the kidney. During such a period, the state of the progress until embolization was controlled by a contrast method.

After 30 days, the kidney was delivered and examined by anatomy. As a result, the embolization of arteria had been conducted well and no re-passing through arteria nor disruption of bloodvessel wall took place in each case.

Then, each slurry was injected onto peritoneum of mouse in an amount of 2 mg/kg in terms of the gelatin particles to conduct a toxicity test. After one week, when subjected to anatomical examination, the particles were collected in the peritoneum and no acute toxicity was observed. Each slurry showed negative when a pre-gel test using pyrogen was conducted.

## Claims

1. A process for producing gelatin spherical gels which comprises dispersing an aqueous solution of gelatin and an aliphatic dial or a water-soluble polyvalent epoxide in a dispersing medium obtained by dissolving water-insoluble ethyl cellulose in a non-polar alicyclic hydrocarbon solvent which is not miscible with water, and conducing a crosslinking reaction.

2. A process according to Claim 1, wherein the alicyclic hydrocarbon is at least one member selected from cyclopentane, cyclohexane, cycloheptane, methylcyclohexane, cyclooctane and decalin.

3. A process according to Claim 1 or Claim 2, wherein the alicyclic hydrocarbon is used together with at least one polar solvent or an aromatic hydrocarbon.

4. A process according to Claim 3, wherein the polar solvent is an ester, a ketone, a halogenated alkane, an ether or an alcohol.

5. A process according to Claim 3, wherein the aromatic hydrocarbon is benzene, toluene, zylene, ethylbenzene, chlorobenzene, dichlorobenzene, bromobenzene or dibromobenzene.

6. A process according to any preceding claim, wherein the water-insoluble ethyl cellulose has the ethoxy group content of 43 to 50% by weight.

7. A crosslinked gelatin spherical particle having a particle size of 50 to 1000 μm for use as an embolic agent.

8. Use of a cross-linked gelatin spherical particle having a particle size of 50 to 1000 μm for the manufacture of an embolic agent.

9. Use of a cross-linked gelatin spherical particle having a particle size of 100 to 1000 μm in the dry state and a swelling volume of 5 to 100 ml/g measured in an isotonic chloride solution, for the manufacture of an embolic agent.

10. An embolic agent, which is a crosslinked gelatin spherical particle having a particle size of 100 to 1000 μm in dry state and a swelling volume of 5 to 100 ml/g measured in an isotonic sodium chloride solution.

## Ansprüche

1. Verfahren zur Herstellung von kugelförmigen Gelatinegelen, dadurch gekennzeichnet, daß man eine wäßrige Lösung von Gelatine und einem aliphatischen Dial oder einem wasserlöslichen mehrwertigen Epoxid in einem Dispergierungsmedium dispergiert, welches dadurch erhalten worden ist, daß man wasserunlösliche Ethylcellulose in einem nichtpolaren alicyclischen Kohlenwasserstoff-Lösungsmittel, das mit Wasser nicht mischbar ist, auflöst und daß man eine Vernetzungsreaktion durchführt.

2. Verfahren nach Anspruch 1, dadurch gekennzeichnet, daß der alicyclische Kohlenwasserstoff mindestens eine Substanz, ausgenwählt aus Cyclopentan, Cyclohexan, Cycloheptan, Methylcyclohexan, Cyclooctan und Decalin, ist.

3. Verfahren nach Anspruch 1 oder 2, dadurch gekennzeichnet, daß man den alicyclischen Kohlenwasserstoff zusammen mit mindestens einem polaren Lösungsmittel oder einem aromatischen Kohlenwasserstoff verwendet.

4. Verfahren nach Anspruch 3, dadurch gekennzeichnet, daß das polare Lösungsmittel ein Ester, ein Keton, ein halogeniertes Alkan, ein Ether oder ein Alkohol ist.

5. Verfahren nach Anspruch 3, dadurch gekennzeichnet, daß der aromatische Kohlenwasserstoff Benzol, Toluol, Zylol, Ethylbenzol, Chlorbenzol, Dichlorbenzol, Brombenzol oder Dibrombenzol ist.

6. Verfahren nach einem der vorstenhenden Ansprüche, dadurch gekennzeichnet, daß die wasserunloslich Ethylcellulose einen Gehalt an Ethoxygruppen von 43 bis 50 Gew.-% hat.

7. Vernetztes kugelformiges Gelatineteilchen mit einer teilchengroße von 50 bis 1000 μm zur Verwendung als embolisches Mittel.

8. Die Verwendung eines vernetzten kugelförmigen Gelatineteilchens mit einer Teilchengröße von 50 bis 1000 μm für die Herstellung eines embolischen Mittels.

9. Die Verwendung eines vernetzten kugelförmigen Gelatineteilchens mit einer Teilchengröße von 100 bis 1000 μm im trockenen Zustand und einem Quellvolumen von 5 bis 100 ml/g gemessen in einer isotonischen Natriumchloridlösung für die Herstellung eines embolischen Mittels.

10. Embolisches Mittel, mämlich ein vernetztes kugelförmiges Gelatineteilchen mit einer Teilchengröße von 100 bis 1000 μm in trockenen Zustand und einem Quellvolumem von 5 bis 100 ml/g, gemessen in einer isotonischen Natriumchloridlösung.

## Revendications

1. Procédé de production de gels sphériques de gélatine, qui consiste à disperser une solution aqueuse de gélatine et un dial aliphatique ou un époxyde polyvalent hydrosoluble dans un milieu de dispersion qu'on obtient en dissolvant une éthylcellulose non hydrosoluble dans un solvant hydrocarboné alicyclique non polaire qui n'est pas miscible avec l'eau et à effectuer une réaction de réticulation.

2. Procédé selon la revendication 1, dans lequel l'hydrocarbure alicyclique est au moins un composé choisi parmi le cyclopentane, le cyclohexane, le cycloheptane, le méthylcyclohexane, le cyclo-octane et la décaline.

3. Procédé selon la revendication 1 ou 2, dans lequel on utilise l'hydrocarbure alicyclique conjointement avec au moins un solvant polaire ou un hydrocarbure aromatique.

4. Procédé selon la revendication 3, dans lequel le solvant polaire est un ester, une cétone, un alcane halogéné, un éther ou un alcool.

5. Procédé selon la revendication 3, dans lequel l'hydrocarbure aromatique est le benzène, le toluène, le xylène, l'éthylbenzène, le chlorobenzène, le dichlorobenzène, le bromobenzène ou le dibromobenzène.

6. Procédé selon l'une quelconque des revendications précédentes, dans lequel l'éthylcellulose non hydrosoluble présente une teneur en groupes éthoxy de 43 à 50% en poids.

7. Particule sphérique de gélatine réticulée, ayant une granulométrie de 50 à 1000 μm, destinée à être employée comme agent embolique.

8. Utilisation d'une particule sphérique de gélatine réticulée, ayant une granulométrie de 50 à 1000 μm, pour la fabrication d'un agent embolique.

9. Utilisation d'une particule sphérique de gélatine réticulée, ayant une granulométrie de 100 à 1000 μm à l'état sec et un volume de gonflement de 5 à 100 ml/g, mesuré dans une solution isotonique de chlorure, pour la fabrication d'un agent embolique.

10. Embole, qui est une particule sphérique de gélatine réticulée, ayant une granulométrie de 100 à 1000 μm à l'état sec et un volume de gonflement de 5 à 100 ml/g, mesuré dans une solution isotonique de chlorure de sodium.